# EUROPÄISCHE PATENTSCHRIFT

(11) **EP 0 770 367 B1**
(45) Veröffentlichungstag und Bekanntmachung des Hinweises auf die Patenterteilung: **19.09.2001**
(21) Anmeldenummer: 96250233.2
(22) Anmeldetag: 17.10.1996
(51) Int. Cl.: A61F 2/44

(54) **Zwischenwirbelimplantat**
Intervertebral implant
Implant intervertébral

(30) Priorität: 26.10.1995 DE 19541114
(43) Veröffentlichungstag der Anmeldung: 02.05.1997
(73) Patentinhaber: BIOMET MERCK Deutschland GmbH, 12247 Berlin (DE)
(72) Erfinder: Papavero, Luca, Dr.med., 22529 Hamburg (DE); Kranz, Curt, Dr.-Ing., 10825 Berlin (DE); Steür, Gerd, 10551 Berlin (DE)
(74) Vertreter: Maikowski, Michael, Dipl.-Ing. Dr.

(56) Entgegenhaltungen:
- EP-A- 0 179 695
- WO-A-94/05235

## Beschreibung

Die Erfindung betrifft ein Zwischenwirbelimplantat gemäß dem Oberbegriff des Anspruchs 1.

Aus WO 94/05235 ist ein Zwischenwirbelimplantat bekannt, das den räumlichen Ersatz einer natürlichen Bandscheibe und die Fixierung der dieser Bandscheibe benachbarten Wirbelkörper in fester Lage zueinander ermöglicht.

Eine derartige Stabilisierung ist bei einer geschädigten Bandscheibe von Bedeutung, da bei einer Sinterung des Zwischenwirbelraums oder einer relativen seitlichen Versetzung der Wirbelkörper gegeneinander Nerven und Rückenmark verletzt werden können, was zu neurologischen Ausfällen und starken Schmerzen bei dem Patienten führt.

Das vorbekannte Zwischenwirbelimplantat weist ein im wesentlichen starres Distanzstück auf, daß anstelle der zu ersetzenden natürlichen Bandscheibe operativ zwischen zwei Wirbelkörpern eingesetzt wird und so einen Höhenverlust des Zwischenwirbelraums verhindert.

Zur Fixierung des Distanzstücks sind die mit den Wirbelkörpern im Eingriff stehenden Stirnflächen des Distanzstücks mit einer profilierten Oberfläche versehen. Hierdurch wird die Reibung zwischen Wirbelkörpern und Distanzstück erhöht und so eine Verschiebung des Distanzstücks zwischen den Wirbelkörpern erschwert.

Weiterhin weist das vorbekannte Zwischenwirbelimplantat einen von oben nach unten durchgehenden Hohlkanal auf, der ein Einwachsen natürlichen Knochenmaterials in das Distanzstück und damit ein Zusammenwachsen der beiden benachbarten Wirbelkörper ermöglicht.

Nachteilig bei dem vorbekannten Zwischenwirbelimplantat ist jedoch, daß das Distanzstück die benachbarten Wirbelkörper mechanisch stark belastet. Bei einer Wirbelsäulenbelastung des Prothesenträgers treten an der Verbindungsstelle von Distanzstück und Wirbelkörpern also relativ hohe mechanische Spannungen auf.

Darüberhinaus besteht das Problem, daß die beiden Wirbelkörper durch den Hohlkanal nur relativ langsam zusammenwachsen.

Der Erfindung liegt deshalb die Aufgabe zugrunde, ein Zwischenwirbelimplantat der eingangs genannten Art zu schaffen, das die Wirbelkörper mechanisch möglichst wenig belastet und ein schnelles Zusammenwachsen der Wirbelkörper bewirkt.

Die Aufgabe wird durch die im kennzeichnenden Teil des Anspruchs 1 enthaltenen Merkmale gelöst.

Die Erfindung schließt die technische Lehre ein, bei einem Zwischenwirbelimplantat die Kontaktfläche zwischen Distanzstück und benachbarten Wirbelkörpern zur Verringerung der von dem Distanzstück auf die Wirbelkörper wirkenden Flächenpressung großflächig zu gestalten und den Hohlkanal so zu formen, daß der Einschub eines das Knochenwachstum fördernden Substratelements in den Hohlkanal möglich ist.

Gemäß der Erfindung ist an den Stirnseiten des Distanzstücks jeweils eine Platte vorgesehen, die im implantierten Zustand die beiden benachbarten Wirbelkörper abstützt.

Diese Platten können wahlweise an das Distanzstück angeformt oder an diesem befestigt, beispielsweise festgeschweißt sein.

Im Bereich des Hohlkanals weisen die beiden Platten jeweils eine Öffnung auf.

Dies ermöglicht das Einwachsen natürlicher Knochensubstanz der beiden Wirbelkörper auf einen Substratträger in den Hohlkanal und damit ein Zusammenwachsen der beiden Wirbelkörper zu einer Einheit.

Das als Träger für die natürliche Knochensubstanz dienende Substratelement ist in seinen Außenabmessungen den Innenabmessungen des Hohlkanals derart angepaßt, daß es diesen im wesentlichen ausfüllt. Insbesondere besteht dieses Substratelement aus einem porösen Material, welches der natürlichen Knochensubstanz so ähnlich ist, daß es von dieser durch Einwachsen durchdrungen wird, so daß ein mechanisch fester Anschluß sichergestellt ist. Dabei sind mit den Maßnahmen der Erfindung die Elastizitäts- und Festigkeitseigenschaften des Implantats an diejenigen des umgebenen Knochenbereichs in der Weise angepaßt, daß dieses im angewachsenen Zustand in isoelastischer Weise dessen Mikrobewegungen folgt, ohne daß es zu wesentlichen Längendifferenzen im Anpassungsbereich kommt, welche zu einem Verlust der angewachsenen Verbundung durch Bruch oder Riß führen könnten. Dabei sind insbesondere auch die - weiter unten beschriebenen Maßnahmen von Bedeutung, welche durch eine entsprechende, quergerichtete Schubkräfte aufnehmende Profilierung für einen festen Halt des Implantats sorgen, wenn es nach dem operativen Einbringen seine vorgesehene Position einnimmt.

Einerseits ist die Masse des Substratelements gering, so daß relativ viel Knochenmasse einwachsen und eine stabile Knochenverbindung zwischen den beiden Wirbelkörpern bilden kann. Andererseits kann das Substratelement den Hohlkanal bei geringer Masse vollständig ausfüllen und somit einen guten Träger für die natürliche Knochenmasse bilden.

Der Außenquerschnitt des Substratelements ist vorzugsweise an den Innenquerschnitt des Hohlkanals in der Weise angepaßt, daß der Hohlkanal mit dem Substratelement eine Spielpassung bildet. Zum einen läßt sich das Substratelement dadurch ohne größeren Kraftaufwand in den Hohlkanal einschieben. Zum anderen wird das Substratelement von dem Hohlkanal geführt und hat damit in dem Distanzstück eine definierte Position.

Das Substratelement ist vorzugsweise vorgefertigt und an das Distanzstück angepaßt. Hierdurch entfällt bei der Implantation das relativ aufwendige Einführen und Anpassen des ansonsten erforderlichen, nicht vorkonfektionierten Trägermaterials. Zum einen wird hierdurch bei der Implantation Zeit gespart, was im Interesse der Patentientenschonung vorteilhaft ist. Zum anderen läßt sich durch die Vorfertigung des Substratelements eine in dem gesamten Volumen des Hohlkanals einheitliche Qualität des Trägermaterials mit einheitlicher Porosität erreichen, was zu einem raschen Zusammenwachsen der beiden Wirbelkörper beiträgt.

In einer vorteilhaften Variante der Erfindung von eigener schutzwürdiger Bedeutung besteht das Substratelement im wesentlichen aus einem aus Rinderspongiosa gebrannten Keramikmaterial. Hierdurch wird zum einen eine gute Biokompatibilität erreicht und zum anderen das Knochenwachstum im Zwischenwirbelraum beschleunigt.

In einer anderen Variante der Erfindung von eigener schutzwürdiger Bedeutung besteht das Substratelement aus einem Material, das knochenwachstumsfördernd wirkt. Hierdurch wird ebenfalls die Ausbildung einer natürlichen Knochenverbindung zwischen den beiden Wirbelkörpern beschleunigt.

Die Fläche der Platten ist gemäß der Erfindung größer als die Querschnittsfläche des Distanzstücks. Die Platten ragen also seitlich über das Distanzstück hinaus.

Hierdurch wird die mechanische Belastung der Wirbelkörper verringert, da bei einer axialen Belastung der Wirbelsäule die von dem Distanzstück auf die Wirbelkörper übertragene Kraft durch die Platten auf eine größere Fläche verteilt wird.

Einerseits wird zwar durch die an der Ober- und Unterseite des Distanzstücks angeordneten großflächigen Platten die auf die Wirbelkörper wirkende Flächenpressung verringert. Andererseits behindern diese Platten jedoch grundsätzlich das Zusammenwachsen der benachbarten Wirbelkörper, da das Wachstum des natürlichen Knochenmaterials von den Platten aufgehalten wird.

Die Platten weisen deshalb gemäß der Erfindung an der Außenseite mindestens einen Durchbruch auf, durch den die natürliche Knochensubstanz an der Außenseite des Distanzstücks hindurchwachsen kann. Nach der Implantation wächst die natürliche Knochensubstanz also ausgehend von den benachbarten Wirbelkörpern zum einen durch den Hohlkanal in dem Distanzstück und zum anderen durch die Durchbrüche in den an den Stirnseiten des Distanzstücks angeordneten Platten außen am Distanzstück vorbei nach Innen in den Zwischenwirbelraum.

Das erfindungsgemäße Zwischenwirbelimplantat kombiniert also die Vorteile eines relativ dünnen, in seinen Abmessungen der ursprünglichen Bandscheibe angepaßten, Distanzstücks mit den Vorteilen großer Kontaktflächen. Einerseits füllt ein dünnes Distanzstück nur einen relativ geringen Volumenanteil des Zwischenwirbelraums aus, so daß relativ viel natürliche Knochensubstanz in den Zwischenwirbelraum einwachsen und eine natürliche Knochenverbindung zwischen den beiden benachbarten Wirbelkörpern bilden kann. Andererseits ist die mechanische Belastung der Wirbelkörper durch das Zwischenwirbelimplantat durch die großflächigen Kontaktflächen zwischen den Wirbelkörpern und dem Zwischenwirbelimplantat relativ gering.

Einerseits muß das Zwischenwirbelimplantat hinreichend starr und fest sein, um während des Zusammenwachsens der beiden benachbarten Wirbelkörper bei einer Belastung der Wirbelsäule eine Relativbewegung der Wirbelkörper zueinander weitgehend zu verhindern, da eine derartige Bewegung das Zusammenwachsen der Wirbelkörper stören würde.

Andererseits sollte das Zwischenwirbelimplantat auch nicht wesentlich starrer sein als natürliche Knochensubstanz. Dies rührt daher, daß die Belastung nach dem Zusammenwachsen der beiden benachbarten Wirbelkörper sowohl- von dem Zwischenwirbelimplantat also auch von der in den Zwischenwirbelraum eingewachsenen natürlichen Knochensubstanz aufgenommen werden soll. Bei einem gegenüber der natürlichen Knochensubstanz wesentlich festeren Zwischenwirbelimplantat wäre dies jedoch nicht gegeben und die Belastung würde nahezu ausschließlich von dem Zwischenwirbelimplantat aufgenommen.

Das Distanzstück weist - wie dargelegt - vorzugsweise eine Nachgiebigkeit auf, die ähnlich der Nachgiebigkeit natürlicher Knochensubstanz ist. In einer bevorzugten Ausführungsform sind deshalb in der Wandung des Distanzstücks seitlich Durchbrüche angebracht, die die Festigkeit des Distanzstücks herabsetzen und damit die Nachgiebigkeit des Zwischenwirbelimplantats erhöhen.

In vorteilhafter Weiterbildung der Erfindung sind die beiden Platten zum Anschluß an die benachbarten Wirbelkörper parallel zueinander ausgerichtet.

Die natürliche Wirbelsäule ist jedoch nicht exakt gerade, sondern gekrümmt. Es ist deshalb oftmals wünschenswert, daß das Zwischenwirbelimplantat entsprechend der natürlichen Krümmung der Wirbelsäule an der Implantationsstelle gekrümmt ist. In einer vorteilhaften Variante der Erfindung sind die beiden Platten deshalb entsprechend der natürlichen Wirbelsäulenkrümmung gegeneinander geringfügig angewinkelt.

Wichtig für das Einwachsen des natürlichen Knochenmaterials in das Zwischenwirbelimplantat ist die freie Zugänglichkeit des Hohlkanals. In einer vorteilhaften weiterbildenden Variante der Erfindung ist das Distanzstück deshalb als Gitterkäfig ausgeführt. Hierdurch wird eine weitgehend ungestörte Zirkulation körpereigener Substanzen im Zwischenwirbelraum und damit ein schnelles Knochenwachstum ermöglicht.

Das erfindungsgemäße Zwischenwirbelimplantat wird anstelle der zu ersetzenden Bandscheibe operativ zwischen zwei Wirbelkörpern eingesetzt und verhindert dann eine Zusammenpressung des Zwischenwirbelraums. Es ist in diesem Zusammenhang außerordentlich wichtig, daß das Zwischenwirbelimplantat zwischen den beiden Wirbelkörpern fixiert ist, da eine Verschiebung des Zwischenwirbelimplantats zu einer Verletzung von Nerven durch das Zwischenwirbelimplantat selbst oder zu einer Zusammenpressung des Zwischenwirbelraums und in Folge ebenfalls zu einer Nervenverletzung führen könnte.

In einer anderen vorteilhaften, die Erfindung weiterbildenden Variante der Erfindung von eigener schutzwürdiger Bedeutung weisen die Platten deshalb an der den Wirbelkörpern zugewandten Seite eine Oberflächenprofilierung auf. Hierdurch wird die Reibung zwischen dem Zwischenwirbelimplantat und den Wirbelkörpern vergrößert und somit eine Verschiebung des Zwischenwirbelimplantats erschwert.

In einer bevorzugten Ausführungsform dieser Variante besteht die Oberflächenprofilierung im wesentlichen aus in Bezug auf die Längsachse des Hohlkanals umlaufenden konzentrischen Rillen. Die Erhebungen zwischen diesen Rillen werden nach der Implantation durch die zwischen den Wirbelkörpern wirkende Belastung in die Wirbelkörper hineingedrückt und fixieren das Zwischenwirbelimplantat dadurch zwischen den beiden Wirbelkörpern. Hierdurch wird das Zwischenwirbelimplantat insbesondere gegenüber Scherbeanspruchungen in Quersichtung fixiert und ein seitliches Herausdrücken des Zwischenwirbelimplantat aus dem Zwischenwirbelraum verhindert. Die durch die Oberflächenprofilierung bewirkte Erhöhung der Belastbarkeit gegenüber Scherbeanspruchungen ist hierbei vorteilhaft unabhängig von der Richtung der Scherbeanspruchung, da die konzentrischen Rillen bzw. Erhebungen der Oberflächenprofilierung in Bezug auf die Längsachse des Hohlkanals umlaufend angeordnet sind und somit keine Vorzugsrichtung aufweisen.

Bei den vorstehend beschriebenen Varianten der Erfindung erfolgt die Verbindung des Zwischenwirbelimplantats mit den benachbarten Wirbelkörpern vorzugsweise kraftschlüssig, beispielsweise durch die zwischen den Wirbelkörpern und der profilierten Plattenoberfläche wirkende Reibung. Bei einer derartigen kraftschlüssigen Verbindung kann eine Verschiebung des Zwischenwirbelimplantats jedoch nicht mit Sicherheit ausgeschlossen werden. Dies ist insbesondere während des Zusammenwachsens der beiden benachbarten Wirbelkörper von Nachteil, da das Zwischenwirbelimplantat eine mechanische Belastung dann allein aufnehmen muß. Darüberhinaus stört eine Bewegung des Zwischenwirbelimplantats im Zwischenwirbelraum das Zusammenwachsen der beiden benachbarten Wirbelkörper.

In einer weiterbildenden Variante der Erfindung von eigener schutzwürdiger Bedeutung sind deshalb Verankerungselemente vorgesehen, die die beiden Platten jeweils formschlüssig mit den benachbarten Wirbelkörpern verbinden. In einer bevorzugten Ausführungsform sind hierzu Knochenschrauben vorgesehen, die durch Bohrungen in den Platten hindurchgesteckt und in die Wirbelkörper eingeschraubt werden.

Durch eine derartige formschlüssige Verbindung ist das Zwischenwirbelimplantat vorteilhaft auch in der Lage eine Biegebelastung der Wirbelsäule aufzunehmen.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
- Figur 1: als bevorzugtes Ausführungsbeispiel der Erfindung ein Zwischenwirbelimplantat im implantierten Zustand im Querschnitt,
- Figur 2a: das Zwischenwirbelimplantat aus Figur 1 in perspektivischer Darstellung sowie
- Figur 2b: das Substratelement des in den Figuren 1 und 2a dargestellten Zwischenwirbelimplantats in perspektivischer Darstellung.

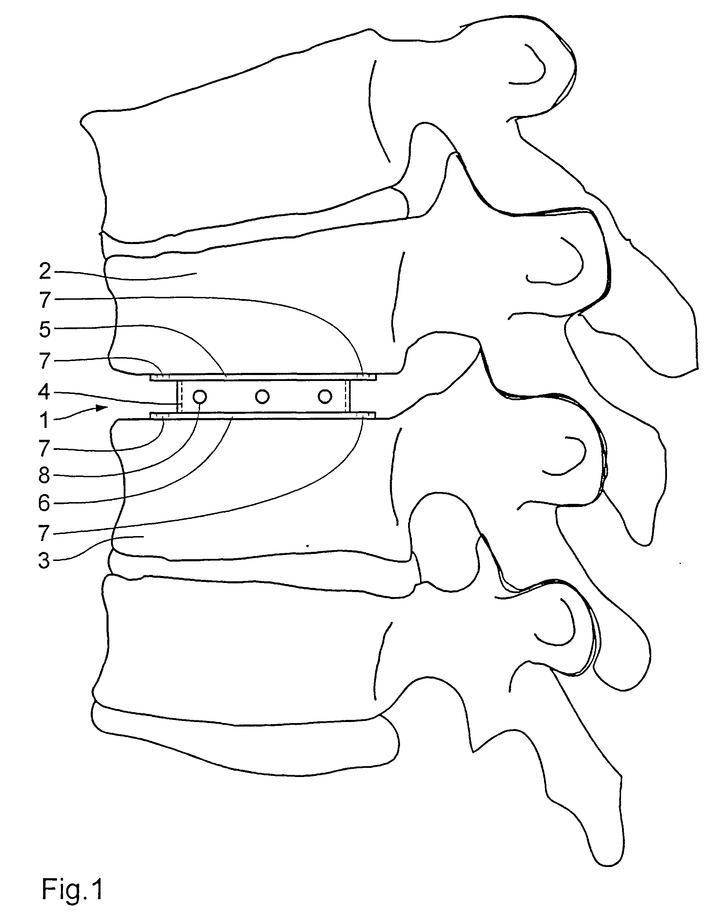

Die in Figur 1 gezeigte Querschnittsdarstellung verdeutlicht zum einen den Aufbau des Zwischenwirbelimplantats 1 und zum anderen die Anordnung des Zwischenwirbelimplantats 1 zwischen zwei Wirbelkörpern 2, 3 zum Ersatz einer natürlichen Bandscheibe.

Das dargestellte Zwischenwirbelimplantat 1 besteht im wesentlichen aus einem Distanzstück 4 zylindrischen Querschnitts mit einem Außendurchmesser von ca. 14 mm und einer Höhe 5 mm. Dieses Distanzstück 4 hat die Aufgabe, an Stelle der natürlichen Bandscheibe die beiden benachbarten Wirbelkörper 2, 3 in einem festen Abstand zueinander zu fixieren, um eine Zusammenpressung des Zwischenwirbelraums mit der Gefahr von Nervenschädigungen zu verhindern.

An dieses Distanzstück 4 ist zur Verbindung des Zwischenwirbelimplantats 1 mit den Wirbelkörpern 2, 3 an der Oberseite und an der Unterseite jeweils eine rechteckige Platte 5, 6 mit einer Länge von 17 mm und einer Breite von 15 mm angeformt. Da die Fläche der Platten 5, 6 größer ist als die Querschnittsfläche des Distanzstücks 4 wird die bei einer Belastung des implantierten Zwischenwirbelimplantats 1 an der Verbindungsstelle auf die Wirbelkörper 2, 3 wirkende Flächenpressung verringert, was zu einer Schonung der Wirbelkörper 2, 3 beiträgt.

Die beiden Platten 5, 6 sind hierbei parallel angeordnet, so daß die beiden der zu ersetzenden Bandscheibe benachbarten Wirbelkörper 2, 3 ohne Krümmung miteinander verbunden werden.

In dem Distanzstück 4 ist ein von oben nach unten durchgehender Hohlkanal mit zylindrischem Querschnitt angeordnet, der ein Einwachsen natürlichen Knochenmaterials und damit den Aufbau einer natürlichen Knochenverbindung zwischen den beiden benachbarten Wirbelkörpern 2, 3 ermöglicht. Die beiden benachbarten Wirbelkörper 2, 3 bilden dann im Endstadium eine feste Knocheneinheit, so daß eine mechanische Belastung der Wirbelsäule nach dem Zusammenwachsen der beiden Wirbelkörper 2, 3 teilweise von dem Zwischenwirbelimplantat 1 und teilweise von der natürlichen Knochenverbindung aufgenommen wird.

Damit die Platten 5, 6 das Einwachsen des natürlichen Knochenmaterials in das Distanzstück 4 nicht behindern, weisen diese mittig eine kreisförmige Öffnung auf, die den gesamten Querschnitt des Hohlkanals freigibt.

Darüberhinaus sind an der Außenseite der Platten 5, 6 jeweils in den Ecken mehrere Durchbrüche 7 angebracht, die das Einwachsen natürlichen Knochenmaterials an der Außenseite des Distanzstücks 4 ermöglichen.

Die natürliche Knochensubstanz wächst also ausgehend von den beiden benachbarten Wirbelkörpern 2, 3 zum einen durch den Hohlkanal in dem Distanzstück 4 und zum anderen durch die Durchbrüche 7 in den Platten 5, 6 an der Außenseite des Distanzstücks 4 nach innen in den Zwischenwirbelraum. Das Zusammenwachsen der beiden benachbarten Wirbelkörper 2, 3 wird deshalb durch die dazwischen angeordneten Platten 5, 6 nur unwesentlich behindert.

Weiterhin sind in der Seitenwand des Distanzstücks 4 weitere kreisförmige Durchbrüche 8 mit einem Durchmesser von ca. 3 mm angeordnet, die ein freies Zirkulieren von Körperflüssigkeiten im Zwischenwirbelraum ermöglichen, was ebenfalls zu einem raschen Zusammenwachsen der beiden benachbarten Wirbelkörper 2, 3 beiträgt.

Darüberhinaus erhöhen diese Durchbrüche 8 die Nachgiebigkeit des Distanzstücks 4. Dies ist sinnvoll, da die mechanische Belastung nach dem Zusammenwachsen der beiden benachbarten Wirbelkörper 2, 3 gemeinsam von dem Zwischenwirbelimplantat 1 und der in das Zwischenwirbelimplantat 1 eingewachsenen natürlichen Knochensubstanz aufgenommen werden soll.

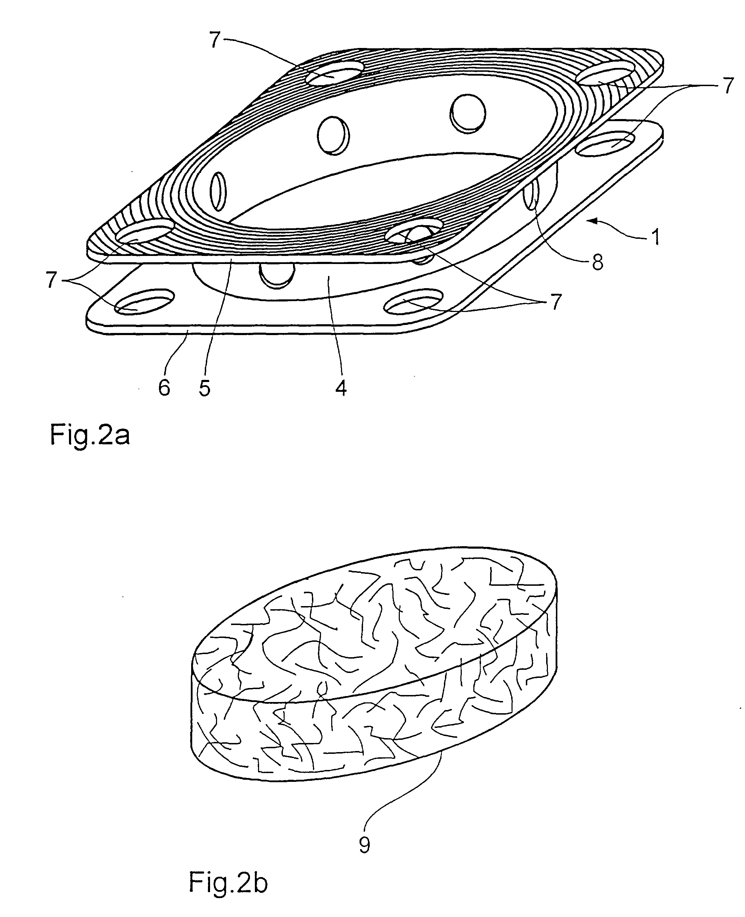

Weiterhin ist aus der in Figur 2a gezeigten perspektivischen Darstellung des Zwischenwirbelimplantats 1 ersichtlich, daß die Platten 5, 6 an der den Wirbelkörpern zugewandten Seite eine Oberflächenprofilierung in Form von umlaufenden Rillen aufweisen.

Die Erhebungen zwischen diesen Rillen werden nach der Implantation infolge der zwischen den Wirbelkörpern wirkenden Belastung in die Wirbelkörper hineingedrückt und verhindern eine seitliche Verschiebung des Zwischenwirbelimplantats 1. Die bei einer Scherbeanspruchung der Wirbelsäule an der Kontaktfläche von Zwischenwirbelimplantat 1 und Wirbelkörpern auftretenden Scherkräfte werden somit von der mit den Wirbelkörpern im Eingriff stehenden Oberflächenprofilierung aufgenommen.

Aus Figur 2a ist ebenfalls ersichtlich, daß die Platten 5, 6 mittig eine kreisförmige Öffnung aufweisen, die den gesamten Querschnitt des in dem Distanzstück 4 verlaufenden Hohlkanals freigibt. Der Querschnitt dieses Hohlkanals ist entlang der Längsachse einheitlich. Hierdurch kann ein als Träger für die natürliche Knochensubstanz dienendes tablettenförmiges Substratelement auf einfache Weise in den Hohlkanal hineingeschoben werden.

Weiterhin wird die Anordnung der Durchbrüche 7 verdeutlicht, die jeweils in den Ecken der Platten 5, 6 angeordnet sind und ein Einwachsen natürlicher Knochensubstanz in den Zwischenwirbelraum an der Außenseite des Distanzstücks 4 ermöglichen.

Das Substratelement 10 ist detailliert in Figur 2b dargestellt und besteht im wesentlichen aus einem porösen Keramikmaterial. Durch die Porösität des Substratelements 10 ist einerseits das tatsächliche Volumen des Einsatzstücks 10 relativ gering, so daß in den Hohlkanal relativ viel natürliche Knochensubstanz einwachsen kann. Andererseits füllt das Substratelement 10 den Hohlkanal vollständig aus, so daß im gesamten Volumen des Hohlkanals ein Träger für die natürliche Knochensubstanz zur Verfügung steht. Durch die Verwendung eines Trägers für die natürliche Knochensubstanz anstelle eines leeren Hohlkanals wird das Knochenwachstum beschleunigt und damit ein schnelles Zusammenwachsen der benachbarten Wirbelkörper erreicht.

Das Substratelement 10 besteht im wesentlichen aus einem aus Rinderspongiosa gebrannten Keramikmaterial. Hierdurch wird zum einen eine gute Biokompatibilität erreicht und zum anderen das Knochenwachstum positiv beeinflußt. Darüberhinaus sind in das Substratelement 10 nach dem Brennen der Keramik knochenwachstumsfördernde Substanzen eingebracht, die das Zusammenwachsen der beiden Wirbelkörper weiter beschleunigen.

Die Form des Substratelements 10 ist zylindrisch, um ein Einschieben in den Hohlkanal des Zwischenwirbelimplantats zu ermöglichen. Der Querschnitt des Substratelements ist deshalb an den Querschnitt des Hohlkanals angepaßt.

Die Erfindung beschränkt sich in ihrer Ausführung nicht auf die vorstehend angegebenen bevorzugten Ausführungsbeispiele. Vielmehr ist eine Anzahl von Varianten denkbar, welche von der dargestellten Lösung auch bei grundsätzlich anders gearteten Ausführungen Gebrauch macht.

## Patentansprüche

1. Zwischenwirbelimplantat zum Einfügen zwischen benachbarte Wirbelkörper (2, 3) mit einem einen durchgehenden Hohlkanal zur Aufnahme natürlicher Knochensubstanz aufweisenden Distanzstück (4),
**dadurch gekennzeichnet,**
daß an den Stirnseiten des Distanzstücks (4) zum Anschluß an die benachbarten Wirbelkörper (2, 3) jeweils eine sich in der Stirnebene erstreckende flanschartige Platte (5, 6) vorgesehen ist,
daß die Platten (5, 6) zur Verringerung der von dem Distanzstück (4) auf die Wirbelkörper (2, 3) ausgeübten Flächenpressung das Distanzstück (4) mindestens bereichsweise seitlich überragen,
daß mindestens eine Platte (5, 6) im Bereich des Hohlkanals zum Einführen eines als Träger für die natürliche Knochensubstanz dienenden Substratelements (9) in den Hohlkanal eine Öffnung aufweisen,
daß die Platten (5, 6) in dem das Distanzstück (4) überragenden Bereich mindestens einen Durchbruch (7) aufweisen.

2. Zwischenwirbelimplantat nach Anspruch 1, **dadurch gekennzeichnet, daß** das Distanzstück (4) im wesentlichen rohrförmig ausgestalttet ist und einen in Längsrichtung im wesentlichen konstanten Innenquerschnitt aufweist.

3. Zwischenwirbelimplantat nach Anspruch 1 oder 2, **dadurch gekennzeichnet, daß** der Innenquerschnitt des Hohlkanals an den Außenschnitt des Substratelements (9) angepaßt ist.

4. Zwischenwirbelimplantat nach einem der vorhergehenden Ansprüche, **dadurch gekennzeichnet, daß** der Innenquerschnitt des Hohlkanals im wesentlichen kreisförmig ist.

5. Zwischenwirbelimplantat nach einem der vorhergehenden Ansprüche, **dadurch gekennzeichnet, daß** das Distanzstück (4) im wesentlichen starr ist.

6. Zwischenwirbelimplantat nach einem der Ansprüche 1 bis 4, **dadurch gekennzeichnet, daß** das Distanzstück (4) eine Nachgiebigkeit aufweist, die im wesentlichen derjenigen von natürlicher Knochensubstanz entspricht.

7. Zwischenwirbelimplantat nach Anspruch 6, **dadurch gekennzeichnet, daß** das Distanzstück (4) zur Erhöhung der Nachgiebigkeit in radialer Richtung mindestens einen Durchbruch (8) aufweist.

8. Zwischenwirbelimplantat nach Anspruch 7, **dadurch gekennzeichnet, daß** das Distanzstück (4) zur Förderung des Hineinwachsens natürlicher Knochensubstanz als Gitterkäfig ausgeführt ist.

9. Zwischenwirbelimplantat nach einem der vorhergehenden Ansprüche, **dadurch gekennzeichnet, daß** die beiden Platten (5, 6) im wesentlichen parallel gerichtet oder entsprechend der natürlichen Wirbelsäulenkrümmung geringfügig gegeneinander angewinkelt sind.

10. Zwischenwirbelimplantat nach einem der vorhergehenden Ansprüche, **dadurch gekennzeichnet, daß** als Werkstoff Titan oder eine Titanlegierung vorgesehen ist.

11. Zwischenwirbelimplantat nach einem der vorhergehenden Ansprüche mit einem Substratelement, **dadurch gekennzeichnet, daß** das Substratelement (9) im wesentlichen aus einem porösen Material besteht.

12. Zwischenwirbelimplantat nach einem der vorhergehenden Ansprüche mit einem Substratelement **dadurch gekennzeichnet, daß** das Substratelement (9) im wesentlichen aus aus Rinderspongiosa gebrannter Keramik besteht.

13. Zwischenwirbelimplantat nach einem der vorhergehenden Ansprüche mit einem Substratelement, **dadurch gekennzeichnet, daß** das Substratelement (9) knochenwachstumsfördernde Substanzen aufnehmen kann.

14. Zwischenwirbelimplantat nach einem der vorhergehenden Ansprüche, **dadurch gekennzeichnet, daß** mindestens eine der Platten (5, 6) an der einem Wirbelkörper (2, 3) zugewandten Seite eine Oberflächenprofilierung aufweist.

15. Zwischenwirbelimplantat nach Anspruch 14, **dadurch gekennzeichnet, daß** die Oberflächenprofilierung im wesentlichen aus mehreren ringförmig umlaufenden Rillen besteht.

16. Zwischenwirbelimplantat nach einem der vorhergehenden Ansprüche, **gekennzeichnet durch** Verankerungselemente zur formschlüssigen Verbindung der Platten (5, 6) mit den Wirbelkörpern (2, 3).

17. Zwischenwirbelimplantat nach Anspruch 16, **dadurch gekennzeichnet, daß** die Verankerungselemente Knochenschrauben sind.

## Claims

1. Intervertebral implant for insertion between adjoining vertebrae (2, 3) with a spacer member (4) having a continuous hollow channel for holding natural bone matter,
**characterised in that**
on the end sides of the spacer member (4) for connecting with the adjoining vertebra (2, 3) there is a flange-like plate (5, 6) which extends in the end plane,
that the plates (5, 6) project laterally at least in some areas past the spacer member (4) in order to reduce the surface pressure exerted by the spacer member (4) on the vertebrae (2, 3),
that at least one plate (5, 6) has an opening in the region of the hollow channel for inserting into the hollow channel a substrate element (9) which serves as carrier for the natural bone matter,
that the plates (5, 6) have at least one aperture (7) in the area projecting beyond the spacer member (4).

2. Intervertebral implant according to claim 1 **characterised in that** the spacer member (4) is formed substantially tubular and has an internal cross-section which is substantially constant in the longitudinal direction.

3. Intervertebral implant according to claim 1 or 2 **characterised in that** the internal cross-section of the hollow channel matches the external section of the substrate element (9).

4. Intervertebral implant according to one of the preceding claims, **characterised in that** the internal cross-section of the hollow channel is substantially circular.

5. Intervertebral implant according to one of the preceding claims **characterised in that** the spacer member (4) is substantially rigid.

6. Intervertebral implant according to one of claims 1 to 4 **characterised in that** the spacer member (4) has a pliability which corresponds substantially to that of natural bone matter.

7. Intervertebral implant according to claim 6 **characterised in that** the spacer member (4) has at least one aperture (8) for increasing the pliability in the radial direction.

8. Intervertebral implant according to claim 7 **characterised in that** the spacer member (4) is formed as a grid cage to promote the growth of natural bone matter into same.

9. Intervertebral implant according to one of the preceding claims **characterised in that** the two plates (5, 6) are directed substantially parallel to or are angled slightly relative to each other corresponding to the natural curvature of the spine.

10. Intervertebral implant according to one of the preceding claims **characterised in that** titanium or a titanium alloy is used as the material.

11. Intervertebral implant according to one of the preceding claims with a substrate element **characterised in that** the substrate element (9) consists substantially of a porous material.

12. Intervertebral implant according to one of the preceding claims with a substrate element **characterised in that** the substrate element (9) consists substantially of ceramic fired with cow spongiosa.

13. Intervertebral implant according to one of the preceding claims with a substrate element **characterised in that** the substrate element (9) can contain bone growth promoting substances.

14. Intervertebral implant according to one of the preceding claims **characterised in that** at least one of the plates (5, 6) has a surface profile on the side facing a vertebra (2, 3).

15. Intervertebral implant according to claim 14 **characterised in that** the surface profile consists substantially of several circular circumferential grooves.

16. Intervertebral implant according to one of the preceding claims **characterised by** anchorage elements for the positive locking connection of the plates (5, 6) with the vertebrae (2, 3).

17. Intervertebral implant according to claim 16 **characterised in that** the anchorage elements are bone screws.

## Revendications

1. Implant intervertébral conçu pour être interposé entre des vertèbres voisines (2, 3), muni d'une pièce d'espacement (4) présentant un canal creux ininterrompu pour recevoir de la substance osseuse naturelle,
**caractérisé par** le fait
qu'une plaque (5, 6) du type bride, s'étendant dans le plan frontal, est respectivement prévue aux faces extrêmes de la pièce d'espacement (4) pour le rattachement aux vertèbres voisines (2, 3) ;
par le fait que les plaques (5, 6) font saillie latéralement au-delà de la pièce d'espacement (4), au moins par zones, en vue de réduire la pression superficielle exercée sur les vertèbres (2, 3) par ladite pièce d'espacement (4) ;
par le fait qu'au moins une plaque (5, 6) comporte, dans la région du canal creux, une ouverture destinée à l'insertion, dans ledit canal creux, d'un élément substrat (9) servant de support affecté à la substance osseuse naturelle ;
par le fait que les plaques (5, 6) possèdent au moins une perforation (7) dans la région dépassant de la pièce d'espacement (4).

2. Implant intervertébral selon la revendication 1,
**caractérisé par** le fait que la pièce d'espacement (4) est pour l'essentiel de configuration tubulaire, et présente une section transversale intérieure sensiblement constante dans le sens longitudinal.

3. Implant intervertébral selon la revendication 1 ou 2, **caractérisé par** le fait que la section transversale intérieure du canal creux est adaptée à la découpe extérieure de l'élément substrat (9).

4. Implant intervertébral selon l'une des revendications précédentes, **caractérisé par** le fait que la section transversale intérieure du canal creux est pour l'essentiel circulaire.

5. Implant intervertébral selon l'une des revendications précédentes, **caractérisé par** le fait que la pièce d'espacement (4) est pour l'essentiel rigide.

6. Implant intervertébral selon l'une des revendications 1 à 4, **caractérisé par** le fait que la pièce d'espacement (4) possède une souplesse élastique correspondant, pour l'essentiel, à celle d'une substance osseuse naturelle.

7. Implant intervertébral selon la revendication 6,
**caractérisé par** le fait que la pièce d'espacement (4) présente au moins une perforation (8), en vue d'accroître la souplesse élastique dans le sens radial.

8. Implant intervertébral selon la revendication 7,
**caractérisé par** le fait que la pièce d'espacement (4) est réalisée sous la forme d'une cage en treillis, pour favoriser la croissance interne d'une substance osseuse naturelle.

9. Implant intervertébral selon l'une des revendications précédentes, **caractérisé par** le fait que les deux plaques (5, 6) sont orientées pour l'essentiel parallèlement, ou sont légèrement coudées l'une vers l'autre d'une manière correspondant à la courbure naturelle de la colonne vertébrale.

10. Implant intervertébral selon l'une des revendications précédentes, **caractérisé par** le fait que du titane ou un alliage de titane est prévu en tant que matériau.

11. Implant intervertébral selon l'une des revendications précédentes, pourvu d'un élément substrat, **caractérisé par** le fait que ledit élément substrat (9) consiste, pour l'essentiel, en un matériau poreux.

12. Implant intervertébral selon l'une des revendications précédentes, poùrvu d'un élément substrat, **caractérisé par** le fait que ledit élément substrat (9) consiste, pour l'essentiel, en une céramique calcinée à partir d'une matière spongieuse d'un vache.

13. Implant intervertébral selon l'une des revendications précédentes, pourvu d'un élément substrat, **caractérisé par** le fait que ledit élément substrat (9) peut recevoir des substances favorisant la croissance osseuse.

14. Implant intervertébral selon l'une des revendications précédentes, **caractérisé par** le fait qu'au moins l'une des plaques (5, 6) présente un profilage de surface sur le côté tourné vers une vertèbre (2, 3).

15. Implant intervertébral selon la revendication 14,
**caractérisé par** le fait que le profilage de surface est constitué, pour l'essentiel, de plusieurs rainures à étendue périphérique annulaire.

16. Implant intervertébral selon l'une des revendications précédentes, **caractérisé par** des éléments d'ancrage en vue de la liaison, par concordance de formes, entre les plaques (5, 6) et les vertèbres (2, 3).

17. Implant intervertébral selon la revendication 16,
**caractérisé par** le fait que les éléments d'ancrage sont des vis de chirurgie osseuse.
